Europäisches Patentamt

**European Patent Office**

**Office européen des brevets**

(11) Numéro de publication: **0 233 115**
A1

## (12) DEMANDE DE BREVET EUROPEEN

(21) Numéro de dépôt: **87400196.9**

(51) Int. Cl.⁴: **A 61 M 5/14**

(22) Date de dépôt: **29.01.87**

(30) Priorité: **03.02.86 FR 8601438**

(43) Date de publication de la demande: **19.08.87**
**Bulletin 87/34**

(84) Etats contractants désignés: **AT BE CH DE ES FR GB GR IT LI LU NL SE**

(71) Demandeur: **BERTIN & CIE, Zone Industrielle Boîte postale 3, F-78373 Plaisir Cédex (FR)**

(72) Inventeur: **Weber, Jean-Luc Michel, Lotissement de Craponne, F-13300 Salon de Provence (FR)**
Inventeur: **Confort, Sylviane Marguerite Marie épouse Gouny, 13 rue de la Guadeloupe, F-13006 Marseille (FR)**

(74) Mandataire: **Colas, Jean-Pierre et al, Cabinet de Boisse 37, avenue Franklin D. Roosevelt, F-75008 Paris (FR)**

(54) **Procédé et système de surveillance à distance d'au moins un poste de perfusion.**

(57) Le système de surveillance comprend une ligne électrique (4) de transmission d'informations numériques, au moins un poste de perfusion (P1-Pn) comportant un débitmètre (3) et un module (M1-Mn) de codage et de transmission sous forme numérique sur la ligne (4) de l'information de débit instantané délivrée par le débitmètre (3), et une unité centrale (UC) de traitement de ladite information, connectée à ladite ligne (4) par l'intermédiaire d'un module de décodage (Muc).

L'unité centrale comporte au moins un écran d'affichage sur lequel s'affichent sous forme analogique et alphanumérique les grandeurs représentatives de l'état du poste de perfusion.

Application à la surveillance de perfusions en milieu hospitalier. ·

ACTORUM AG

## Procédé et système de surveillance à distance d'au moins un poste de perfusion.

L'invention concerne un procédé et un système de surveillance à distance d'au moins un poste de perfusion.

Différentes solutions sont utilisées en milieu hospitalier pour injecter lentement des liquides par voie parentérale, à savoir l'injection par seringue électrique (pousse-seringue), l'injection par pompe à perfusion programmée et la perfusion par gravité.

Les deux premières techniques mentionnées ci-dessus font appel à des matériels situés dans la chambre du patient, au pied du lit, permettant d'assurer un débit constant du produit à injecter avec une grande précision de l'ordre de 1/1000 à 1/10000. Ces appareils sont utilisés en secteur de soins intensifs ou lorsqu'il apparaît nécessaire de contrôler parfaitement le débit de produit délivré dans l'organisme. Ils nécessitent, en particulier, un cathétérisme veineux afin de s'assurer d'une parfaite perméabilité de la voie. Ces matériels sont bien entendu coûteux et ne sont utilisés que dans un nombre limité de cas.

C'est la raison pour laquelle, environ 80 % des perfusions pratiquées en Europe font appel à la technique de la perfusion par gravité. Ce type de perfusion présente l'avantage d'un matériel peu coûteux (bouteilles, tubulures, aiguilles) et d'une facilité de mise en place, au détriment toutefois d'une bonne maîtrise de l'écoulement qui nécessite une surveillance constante et attentive et, par conséquent, des visites fréquentes du personnel responsable auprès des patients perfusés.

La mise en place de la perfusion par gravité doit être initialisée par ce responsable, en estimant de manière empirique un débit de perfusion qui permettra au patient d'absorber la totalité de la perfusion pendant un laps de temps fixé par le médecin. Ce débit est généralement corrigé en plus ou en moins par le responsable, quelques dizaines de minutes après la mise en place de la perfusion afin de s'approcher du débit optimal. Pendant toute la durée de la perfusion, le responsable contrôle si celle-ci s'effectue correctement et surveille qu'il n'y a pas d'interruption de circuit, éventuellement due au patient.

Le responsable est généralement chargé de surveiller plusieurs perfusions et, dans les services importants, plusieurs personnes peuvent être consacrées uniquement à la visite fréquente des chambres des patients perfusés.

L'invention vise à fournir un procédé et un système de surveillance à distance qui permettent à un responsable unique d'évaluer à tout instant, à partir d'une salle de contrôle, le bon fonctionnement d'un ou plusieurs postes de perfusion situés en des lieux différents et d'alerter ce responsable dans un délai suffisant en cas d'anomalie dans le déroulement de la perfusion. Un but plus particulier de l'invention est de permettre à ce responsable d'effectuer une surveillance permanente, en temps réel, de l'évolution de la ou des perfusions en cours dont il a la charge. En outre, bien que l'invention vise principalement la surveillance de postes de perfusion par gravité, elle n'est pas limitée à ce type de perfusion et peut s'appliquer également aux techniques de perfusion à débit commandé, notamment celles mentionnées précédemment.

A cet effet, l'invention a pour objet un procédé de surveillance à distance d'au moins un poste de perfusion pour l'injection d'un liquide à un patient, caractérisé en ce que :

- on raccorde chaque poste de perfusion à surveiller à une ligne de transmission de données numériques,
- on affecte à chaque poste de perfusion à surveiller un code d'identification indépendant de sa localisation géographique,
- on mesure à chaque poste sous surveillance le débit d'injection de liquide,
- on envoie périodiquement cette information à une unité centrale de traitement par la ligne de transmission de données numériques, et
- on affiche sur au moins un écran de contrôle une donnée d'identification fonction dudit code ainsi que le débit instantané de liquide à chaque poste de perfusion sous surveillance.

Suivant une autre caractéristique, on mesure le débit instantané par comptage des gouttes à injecter sur de courtes périodes consécutives d'environ 1 minute, on le compare au débit nominal prévu initialement et on affiche le résultat de ladite comparaison.

L'invention a également pour objet un système de surveillance à distance d'au moins un poste de perfusion pour l'injection d'un liquide à un patient, caractérisé en ce qu'il comprend une ligne électrique de transmission d'informations numériques, au moins un poste de perfusion comportant un débitmètre et un module de codage et de transmission sous forme numérique sur la ligne de l'information de débit instantané délivrée par le débitmètre, une unité centrale de traitement de ladite information, connectée à ladite ligne par l'intermédiaire d'un module de décodage, et au moins un écran de contrôle pour l'affichage de données d'identification et de fonctionnement de chaque poste de perfusion sous surveillance.

De préférence, ladite ligne électrique est une ligne de transmission banalisée du réseau de distribution d'énergie électrique.

D'autres caractéristiques et avantages de la présente invention ressortiront de la description qui va suivre d'un mode de sa réalisation donné uniquement à titre d'exemple et illustré par les dessins annexés sur lesquels :

La figure 1 est un schéma simplifié d'un système de surveillance à distance de postes de perfusion par gravité.

La figure 2 est un schéma-bloc de l'unité centrale du système de la figure 1.

La figure 3 est un algorithme du programme principal mis en oeuvre par l'unité centrale de la figure 2.

La figure 4 est un algorithme d'un sous-programme illustrant la gestion des postes de perfusion à partir de l'unité centrale.

La figure 5 est une représentation d'un écran de contrôle tel qu'il apparaît à un responsable de la surveillance de perfusions ; et

La figure 6 montre une variante de présentation des informations de contrôle de perfusion dans une fenêtre de l'écran de contrôle.

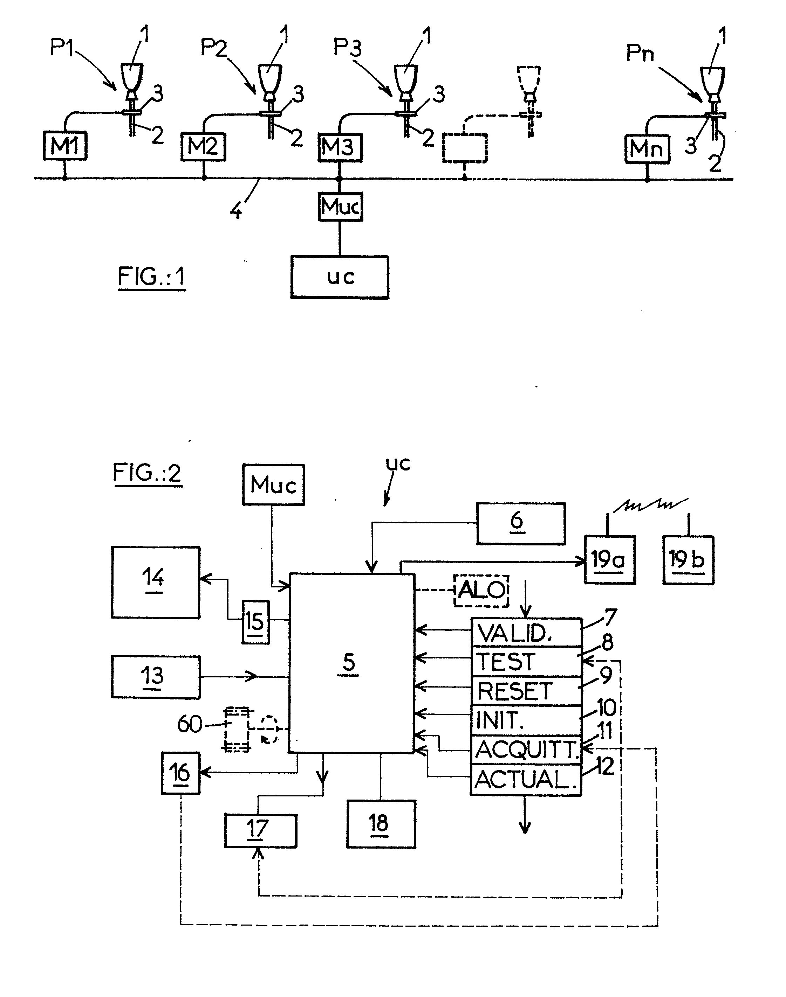

On a représenté sous forme schématique à la figure 1 un système de surveillance, à partir d'une unité centrale UC, d'un certain nombre de postes de perfusion par gravité P1, P2, P3,......Pn. Chaque poste de perfusion comprend un flacon 1 destiné à être rempli du liquide à injecter chez le patient et raccordé à cet effet par une tubulure 2 à une aiguille (non représentée). Sur chaque tubulure 2 est monté un débitmètre 3 qui, dans le cas décrit d'une perfusion par gravité, est constitué par un compte-gouttes tel que par exemple, l'appareil IMED 730 de la Société IMED, U.S.A. Le débitmètre 3 est éventuellement associé à un dispositif d'affichage (non représenté) qui permet de lire le nombre de gouttes par unité de temps directement sur le lieu de la perfusion comme cela est classique. Les débitmètres 3 des postes de perfusion P1 à Pn sont connectés chacun à un module M1 à Mn respectivement qui assure le codage de l'information de nombre de gouttes par unité de temps délivré par le débitmètre 3 correspondant et sa transmission sous forme numérique sur une ligne électrique de transmission 4. De son côté, l'unité centrale UC est raccordée à la ligne de transmission 4 par l'intermédiaire d'un module de décodage Muc. Chaque poste de perfusion P1......Pn possède un numéro d'ordre ou code d'identification qui lui est propre et sera reconnu par l'unité centrale UC.

La ligne électrique de transmission 4 peut faire partie, soit d'un réseau spécialisé pour les transmissions de données, soit d'un réseau de distribution d'énergie électrique continue ou alternative tel que par exemple, le réseau domestique de distribution d'énergie électrique alternative sous basse tension. Le procédé de transmission de données numériques au moyen d'une telle ligne de transmission et des modules associés M1 à Mn et Muc est déjà connu et ne sera pas décrit en détail. On pourra notamment se reporter à la demande de brevet FR-A-2 558 667 qui donne une description détaillée du procédé de transmission, du réseau et des modules associés qui peuvent être utilisés dans le système de contrôle suivant l'invention.

On se reportera maintenant à la figure 2 qui montre les différents composants de l'unité centrale UC organisée autour d'un circuit électronique 5 tel que le circuit EFS-MPU 8 qui fait appel au microprocesseur 16 bits MC 68008 de la Société MOTOROLA. Au circuit 5 sont associés le module de décodage Muc, un interrupteur de marche-arrêt 6, un jeu de six touches lumineuses 7 à 12 dont le rôle sera expliqué ci-après, un clavier numérique 13 pour l'entrée des données, et un écran de visualisation 14 relié au circuit 5 par l'intermédiaire d'un circuit 15 de gestion de graphique tel que le circuit EFS-VIG-1 de la Société THOMSON. Le circuit électronique 5 pilote également un générateur d'alarme sonore 16 présentant une liaison avec la touche 11 d'acquittement pour son activation en cas d'apparition d'une alarme, ainsi qu'un dispositif d'affichage 17, par exemple à cristaux liquides, relié à la touche 8 de test pour afficher un code de contrôle ou d'erreur sur quelques digit en cas d'actionnement de la touche de test. Enfin, une batterie de secours 18 peut être prévue pour assurer une sauvegarde en cas de défaillance de l'alimentation principale du système.

Le cas échéant, le système de surveillance peut être complété par un dispositif d'alarme à distance comprenant un émetteur haute-fréquence de faible portée 19a connecté au circuit électronique 5 et un récepteur 19b destiné à être porté par un responsable de la surveillance des perfusions. Le récepteur 19b peut être équipé d'un transducteur acoustique pour l'émission d'une alarme sonore ainsi que de moyens d'affichage permettant l'identification du poste de perfusion concerné par une alarme.

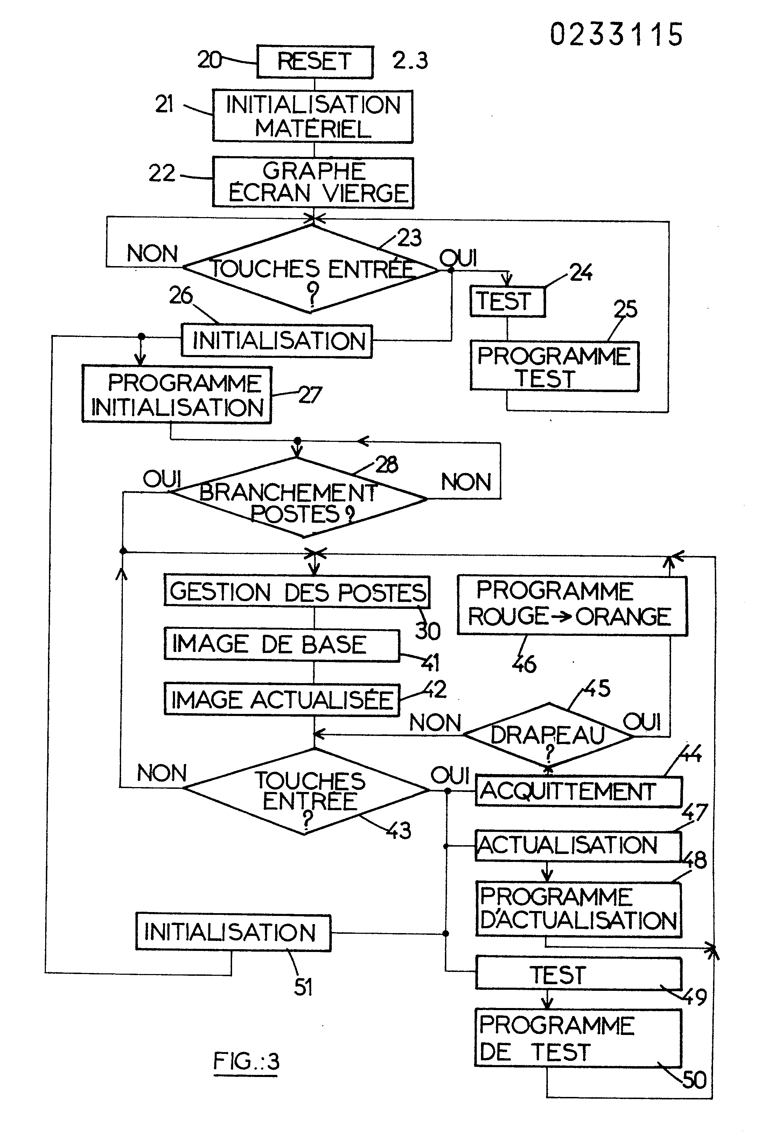

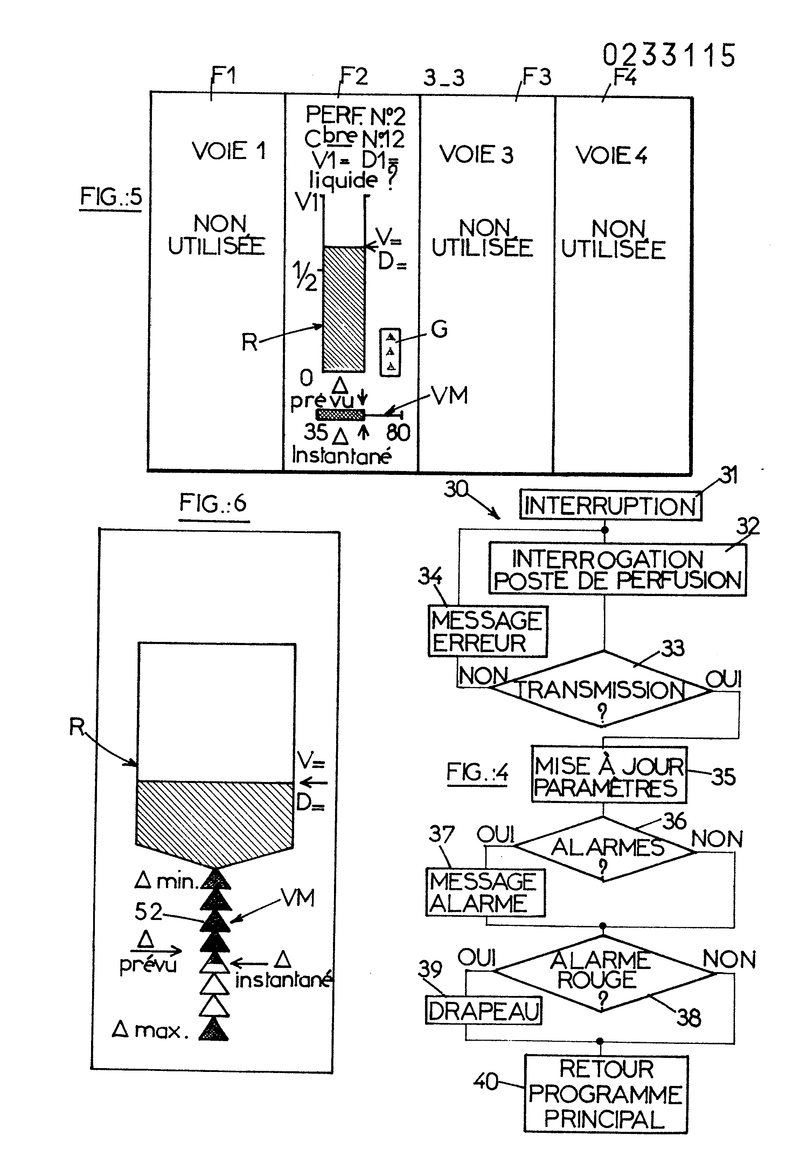

Le fonctionnement de ce dernier sera maintenant décrit en se référant aux algorithmes des figures 3 et 4 ainsi qu'à la figure 5 donnant un exemple d'affichage sur l'écran de visualisation 14 pour quatre postes de perfusion.

Après la mise sous tension du système, l'actionnement de la touche RESET 9 à l'étape 20 conduit à l'étape 21 d'initialisation de la partie matérielle du système et à l'étape 22 d'affichage d'un graphisme d'attente sur l'écran de visualisation 14.

L'étape suivante 23 est un test d'identification de la touche d'entrée actionnée par l'opérateur. Si celui-ci actionne la touche de TEST 8 (étape 24), l'unité centrale déroule un programme 25 pour contrôler le nombre de postes de perfusion P1 à Pn qui sont en service et affiche un code de contrôle sur le dispositif d'affichage 17. Lorsque ce programme de test 25 est terminé, on revient à l'étape 23.

Si, à la suite du test 23, on actionne la touche d'INITIALISATION 10 (étape 26), celle-ci s'allume comme c'est le cas pour l'actionnement des autres touches et l'on passe à l'étape 27 du déroulement du programme d'initialisation.

L'écran de visualisation est alors découpé en quatre fenêtres verticales F1 à F4 dans chacune desquelles apparaît en caractères clignotants le message "perfusion n°". L'opérateur peut alors affecter à chaque fenêtre le numéro de l'un des postes de perfusion P1 à Pn à surveiller en tapant le numéro correspondant sur le clavier numérique 13 et en validant à chaque fois l'instruction introduite grâce à la touche de VALIDATION 7. Ce numéro constitue le code d'identification, qui peut revêtir tout autre forme appropriée, du poste à surveiller. Le processus se reproduit ensuite en séquences pour l'introduction des données relatives au numéro de la chambre du patient, au liquide de perfusion à lui injecter, au volume de liquide à perfuser, et à la durée de perfusion prévue. Dans l'unité centrale est mémorisée une table de conversion qui permet, suivant la nature du liquide perfusé et le nombre de gouttes mesuré par unité de temps, de traduire ce nombre de gouttes en volume de liquide. Lorsque cette séquence d'opérations est achevée, le programme d'initialisation provoque l'affichage dans la fenêtre correspondante de l'écran 14 d'un message indiquant à l'opérateur le débit moyen de perfusion prévu et lui rappelant de brancher le poste de perfusion dont le numéro est affiché sur cette fenêtre. Si ce branchement a déjà été effectué, l'information correspondante est délivrée à l'opérateur sous forme d'une inversion du contraste des fenêtres initialisées par rapport aux fenêtres non initialisées.

On passe ensuite à l'étape 28 où un test sur le branchement des postes de perfusion est effectué. Si ce branchement n'a pas eu lieu, on revient à l'entrée du test 28, tandis que dans le cas contraire, l'opérateur en est averti comme indiqué précédemment par une inversion du contraste de la fenêtre correspondant au poste initialisé. La fenêtre d'un tel poste présente alors l'allure représentée pour la deuxième fenêtre F2 en partant de la gauche sur la figure 5. Cette fenêtre comporte à sa partie supérieure, sous forme de messages affichés en caractères alphanumériques, les données relatives au poste de perfusion concerné, à savoir : le numéro du poste de perfusion, le numéro de la chambre du patient, la nature du liquide de perfusion, le volume à perfuser V1, et la durée D1 de perfusion prévue. D'autre part, cette même fenêtre comporte une représentation graphique d'un tube à essais R dont la largeur représentée est fonction du débit prévu de liquide à injecter, dont la position du niveau est fonction de la durée restante D de la perfusion, et dont la surface délimitée au-dessous de ce niveau est donc fonction du volume V de liquide restant à perfuser. A hauteur de ce niveau est également affichée une flèche à laquelle sont associées les lettres V et D d'identification des deux grandeurs précédentes et leur valeur numérique exprimée, par exemple,

en cm$^3$ pour le volume résiduel de liquide à perfuser et en minutes pour la durée restante. La fenêtre comporte également une représentation graphique G d'un goutte-à-goutte simulant, avec une cadence fonction du débit instantané mesuré, la chute réelle des gouttes s'effectuant au niveau du récipient du poste de perfusion correspondant. Enfin, la fenêtre est complétée par un vu-mètre linéaire VM associant une représentation analogique du débit instantané, par rapport à des valeurs minimale et maximale autorisées, à un affichage numérique de ces mêmes valeurs, la valeur numérique du débit instantané étant adjacente à une flèche coïncidant avec le changement d'aspect des deux segments consécutifs constituant ladite représentation analogique.

Lorsque la réponse au test 28 montre que le branchement des postes de perfusion a bien été effectué, on passe au sous-programme 30 de gestion des postes de perfusion. Ce sous-programme 30 dont l'algorithme est représenté à la figure 4 est initialisé sur une interruption 31. Les interruptions peuvent être générées par l'unité centrale ou par les postes de perfusion eux-mêmes suivant un protocole de gestion des interruptions tel que celui décrit dans la demande de brevet français FR-A-2 558 667 à laquelle on se reportera. On supposera dans la suite que l'interruption provient de l'un des modules M1 à Mn associés aux postes de perfusion, ce qui a pour effet de placer l'unité centrale en état d'interrogation pour recevoir le message émis par ce poste (étape 32). L'étape suivante 33 est un test effectué sur la transmission opérée entre le poste de perfusion et l'unité centrale. Si cette transmission est défaillante, un message d'erreur apparaissant sur le dispositif d'affichage 17 est émis à l'étape 34, après quoi l'on revient à l'étape 32. Si la transmission est effectuée correctement, l'unité centrale procède à la mise à jour des paramètres destinés à être affichés sur l'écran de visualisation 14, dans la fenêtre affectée du numéro correspondant à celui du poste de perfusion ayant effectué la transmission (étape 35). Un test 36 est ensuite effectué pour vérifier si les paramètres mis à jour correspondent à une condition d'émission d'alarme. Si tel est le cas, un message d'alarme est émis à l'étape 37.

Les alarmes émises peuvent avoir un caractère de simple avertissement ou un caractère d'urgence, et elles seront désignées respectivement alarme orange et alarme rouge dans la suite. une condition d'alarme orange provoque le clignotement sur l'écran 14 du paramètre concerné et éventuellement l'allumage d'un voyant d'alarme supplémentaire ALO, et persiste jusqu'à la disparition de sa cause qui peut être :

- un débit instantané mesuré situé hors des limites minimale et maximale ;

- un débit instantané nul pendant un temps inférieur à une durée prédéterminée de, par exemple, 4 minutes, fonction du temps de coagulation du sang ;

- un poste de perfusion débranché pendant un temps inférieur à une durée prédéterminée de, par exemple, 4 minutes, fonction du temps de coagulation du sang ; et

- une situation d'alarme rouge transformée en alarme orange par enfoncement de la touche ACQUITTEMENT par l'opérateur.

Les alarmes rouges se manifestent par un clignotement du paramètre concerné sur l'écran de visualisation 14, le clignotement de la touche d'ACQUITTEMENT 11 et de l'éventuel voyant d'alarme, l'émission d'un signal sonore de type sirène ou message de synthèse vocale par le générateur 16 et l'activation de la touche d'ACQUITTEMENT. Par ailleurs, si le système est équipé du système d'alarme à distance, celui-ci est activé afin de prévenir l'infirmière(s) responsable du déroulement anormal de la perfusion concernée. Celle-ci est avertie par un signal sonore de l'existence d'une anomalie et peut identifier sur son récepteur portable le poste concerné grâce aux moyens d'affichage qui lui indiquent le numéro de la chambre du patient ou toute autre donnée d'identification de la perfusion concernée.

Une alarme rouge est déclenchée lorsque le débit instantané reste nul ou qu'un poste de perfusion demeure débranché pendant un temps supérieur à la durée prédéterminée précitée, ou encore si la mise à jour des paramètres fait apparaître que la durée restante de la perfusion est inférieure à une valeur prédéterminée de, par exemple, 20 minutes.

L'enfoncement de la touche d' ACQUITTEMENT 11 permet de transformer une alarme rouge en alarme orange, c'est-à-dire d'interrompre l'émission du signal sonore au niveau du générateur 16 et du récepteur 19b et de supprimer le clignotement du voyant de la touche. En variante, cette fonction d' ACQUITTEMENT peut être supprimée pour des raisons de sécurité.

La remise en conformité du système est obtenue en supprimant la cause de l'alarme rouge (débit nul ou poste de perfusion débranché), ou en appuyant sur l'une des touches 10 et 12 d' INITIALISATION et d' ACTUALISATION dont les voyants sont également allumés lorsqu'on se trouve dans le délai précité de fin de perfusion.

Après l'étape 37 ou en cas de réponse négative au test 36, on passe au test 38 pour déterminer si les paramètres mis à jour correspondent à une condition d'alarme rouge. Si tel est le cas, il y a émission d'un drapeau en 39 et on retourne ensuite au programme principal (étape 40). Il en va de même si la réponse au test 38 est négative.

Les étapes suivantes 41 et 42 du programme principal consistent à rafraîchir l'image de base et à actualiser les zones variables de cette image, à savoir la fenêtre du poste de perfusion concerné. Un test 43 est ensuite effectué pour déterminer si l'une des touches d'entrée 7 à 12 a été actionnée. Dans la négative, on revient au début du sous-programme 30 de gestion des postes de perfusion. Ce sous-programme se répète pour chacun des postes de perfusion avec un cadencement fonction des interruptions émis par ces derniers, de telle manière qu'une actualisation des paramètres de chacun des postes de perfusion est assurée avec une périodicité de, par exemple, une minute. En d'autres termes, chaque fenêtre de l'écran de visualisation 14 est remise à jour toutes les minutes.

Si la réponse au test 43 est positive, c'est que l'une des touches 7 à 12 a été enfoncée. L'actionnement d'une touche provoque l'émission d'un code particulier reconnu par l'unité centrale. S'il s'agit de la touche d'ACQUITTEMENT 11 (étape 44), un test a lieu en 45 pour déterminer si un drapeau a été émis à l'étape 39 du sous-programme 30. Dans la négative on revient au test 43, tandis que dans l'affirmative un sous-programme de passage d'alarme rouge en alarme orange se déroule. Après ce sous-programme 46, on revient au début du sous-programme 30.

Si l'opérateur a agi sur la touche d'ACTUALISATION 12 (étape 47), il y a déroulement d'un sous-programme d'actualisation 48. Ce sous-programme est similaire au programme d'initialisation et n'entraîne une remise à jour des représentations graphiques dans les fenêtres que lorsque les paramètres qui déterminent ces représentations ont été modifiés. La fin de ce sous-programme 48 ramène au début du sous-programme 30.

En cas d'actionnement de la touche de TEST (étape 49), on passe à un programme de test 50 similaire à celui de l'étape 25, puis on revient au début du sous-programme 30.

Enfin, une action sur la touche d'INITIALISATION 10 ramène au programme 27 d'initialisation (étape 51).

On notera encore que la touche de VALIDATION 7 a uniquement pour fonction de permettre à l'unité centrale d'accepter les valeurs numériques introduites par l'intermédiaire du clavier 13 à l'occasion des programmes d'initialisation 27 ou d'actualisation 48, et n'influence pas le déroulement du programme principal dans les autres cas. De même, on notera qu'un actionnement de la touche d'ACQUITTEMENT 11 ou d'ACTUALISATION 12 à l'occasion du test 23 ramènerait directement à l'entrée de ce test.

On a représenté à la figure 6 une variante de représentation graphique apparaissant dans une fenêtre de l'écran de visualisation 14. Sur cette variante, le récipient R a une forme symbolisée de flacon de perfusion et le vu-mètre VM est disposé verticalement dans le prolongement de ce qui est supposé être l'orifice de ce flacon. Ce vu-mètre VM se présente sous la forme d'une série d'éléments consécutifs de forme triangulaire 52 symbolisant des gouttes. Les éléments d'extrémité 52 correspondant respectivement au débit minimal et au débit maximal acceptable ainsi que les éléments compris entre le débit minimal et le débit instantané mesuré pourront avoir un même aspect, différent de celui des éléments compris entre le débit instantané mesuré et le débit maximal autorisé. Comme dans le cas de la figure 5, des valeurs numériques peuvent être affichées au droit des éléments correspondant respectivement au débit minimal $\Delta$ min, au débit maximal $\Delta$ max, au débit instantané mesuré $\Delta$ instantané, et au débit prévu $\Delta$ prévu.

Il est également possible d'utiliser le vu-mètre VM de la figure 6 pour simuler la chute réelle des gouttes, avec une cadence fonction du débit instantané mesuré.

Il résulte de ce qui précède que le système de surveillance à distance suivant l'invention permet d'assurer un suivi rigoureux des patients perfusés sans nécessiter de déplacements multiples vers la chambre du malade. L'évolution des perfusions peut être surveillée en permanence, les conditions de surveillance pour l'équipe médicale se trouvent facilitées et le délai d'intervention en cas d'anomalie est réduit au plus court. Ce système constitue un progrès décisif pour le suivi des perfusions par gravité, tout en pouvant également être adapté à d'autres types de perfusion, notamment celui mis en oeuvre par pompe programmée.

Afin de tenir compte des conditions d'écoulement hydrodynamique des perfusions propres à chaque service hospitalier (modèle de tubulure, hauteur du flacon par rapport au point d'injection, température ambiante, etc..), une molette graduée 60 située à l'arrière de l'unité centrale permet, lors de sa manipulation, de modifier le coefficient définissant le volume unitaire de base d'une goutte (indépendamment de toute correction liée à la nature du liquide ou à la valeur du débit). Il est ainsi possible d'étalonner le système en fonction des nouvelles habitudes prises dans le service hospitalier concerné (changement de fournisseurs de tubulure par exemple).

Dans un mode préféré de réalisation, le système décrit ne nécessite pas la mise en place d'une infrastructure de connexion supplémentaire grâce à l'utilisation du réseau domestique de distribution d'énergie électrique basse-tension. Par ailleurs, quels que soient les déplacements d'un patient perfusé (changement de lit, changement de chambre), celui-ci reste sous surveillance par simple branchement dans une prise de courant murale quelconque. Le poste de perfusion concerné continue à être identifié par le numéro qui lui est propre (code d'identification) et les données relatives à ce poste s'affichent sur la fenêtre affectée de ce numéro. L'opérateur devra si nécessaire modifier le numéro de chambre apparaissant dans la fenêtre. En complément ou à la place du numéro de chambre, un code d'identification ou le nom du patient peut être affiché. En variante, on peut associer à chaque prise de courant un sous-codeur sur lequel viendra se raccorder le codeur proprement dit. Ce sous-codeur génèrera un code de localisation géographique en complément du code d'identification du poste de perfusion de sorte que l'identification de chaque poste et sa localisation seront effectuées automatiquement par l'unité centrale.

D'autre part, l'utilisation d'un réseau plus étendu, par exemple le réseau commuté des postes et télécommunications, permet d'envisager la télésurveillance à domicile des patients au moyen de ce système. Ce dernier n'est pas limité quant au nombre de postes de perfusion à contrôler, la principale limitation tenant à la capacité du responsable à surveiller un grand nombre de postes. A titre d'exemple, quatre écrans de visualisation divisés chacun en quatre fenêtres verticales permettent à une seule et même personne de surveiller jusqu'à seize postes de perfusion.

On notera enfin que ce système peut également être utilisé pour la télécommande de postes de perfusion en dotant le module Muc de capacité de codage et les modules M1 à Mn de capacité de décodage. Pour une telle télécommande, on peut également faire appel au réseau et au protocole d'échange d'informations entre les périphériques et l'unité centrale qui sont décrits dans la demande de brevet français FR-A-2 558 667 déjà citée. L'unité centrale est alors adaptée pour commander chaque pompe d'un poste de perfusion en fonction de valeurs de consigne.

Par ailleurs, on peut prévoir pour chaque récipient de per-

- 16 -

0233115

fusion 1 un capteur de niveau minimal constitué, par exemple, par une photodiode associée à un photo-transistor, et susceptible de détecter le passage du niveau du liquide par changement d'indice de réfraction et de provoquer le déclenchement de l'alarme de fin de perfusion.

REVENDICATIONS DE BREVET

1. Procédé de surveillance à distance d'au moins un poste de perfusion pour l'injection d'un liquide à un patient, caractérisé en ce que :

- on raccorde chaque poste de perfusion à surveiller à une ligne de transmission de données numériques,

- on affecte à chaque poste de perfusion à surveiller un code d'identification indépendant de sa localisation géographique,

- on mesure à chaque poste (P1, P2 ...Pn) sous surveillance le débit d'injection de liquide,

- on envoie périodiquement cette information à une unité centrale de traitement (UC) par la ligne de transmission de données numériques, et

- on affiche sur au moins un écran de contrôle (14) une donnée d'identification fonction dudit code ainsi que le débit instantané de liquide (Δ) à chaque poste de perfusion sous surveillance.

2. Procédé selon la revendication 1, caractérisé en ce qu'on traite l'information de débit instantané (Δ) afin de remettre à jour périodiquement au moins l'une des grandeurs (V,D) représentatives de l'état de chaque perfusion en cours, comprenant la durée restante (D) de la perfusion et le volume résiduel (V) de liquide à injecter calculés par l'unité centrale à partir du débit instantané mesuré (Δ) et des données initiales de la perfusion comprenant le volume de liquide à injecter (V1), et on affiche chaque grandeur mise à jour sur l'écran de contrôle.

3. Procédé selon la revendication 2, caractérisé en ce que l'on mesure le débit instantané par comptage des gouttes injectées sur de courtes périodes consécutives d'environ une minute, on le compare au débit nominal prévu initialement et on affiche le résultat de ladite comparaison.

4. Procédé selon l'une quelconque des revendications 2 et 3, caractérisé en ce que l'on déclenche une première alarme en cas de détection de l'une au moins des conditions suivantes :
- le débit instantané est respectivement inférieur ou supérieur à des valeurs minimale et maximale prédéterminées,
- le débit instantané est nul,
- un poste de perfusion est déconnecté de l'unité centrale.

5. Procédé selon la revendication 4, caractérisé en ce que l'on déclenche une deuxième alarme en cas de détection de l'une au moins des conditions suivantes :
- le débit instantané reste nul pendant une durée supérieure à une première limite prédéterminée,
- un poste de perfusion est déconnecté de l'unité centrale pendant une durée supérieure à une deuxième limite prédéterminée,
- la durée restante calculée de la perfusion est inférieure à une troisième limite prédéterminée.

6. Procédé selon la revendication 5, caractérisé en ce que les première et seconde limites prédéterminées sont fonction du temps de coagulation du sang.

7. Procédé selon l'une quelconque des revendications 5 et 6, caractérisé en ce qu'on passe de la deuxième à la première alarme au moyen d'une commande manuelle déclenchée par l'intermédiaire d'un terminal (11) associé à l'unité centrale.

8. Procédé selon l'une quelconque des revendications 5 à 7, caractérisé en ce que la première alarme comprend l'allumage d'au moins un voyant lumineux (ALO) et la deuxième alarme comprend le clignotement d'un autre voyant (11) et l'émission d'un signal sonore.

9. Procédé selon l'une quelconque des revendications 1 à 8, caractérisé en ce que l'affichage des grandeurs représentatives de l'état dudit poste comprend une représentation graphique (R) d'un récipient dont la largeur représentée est fonction du débit prévu de liquide à injecter, dont la position du niveau est fonction de la durée restante (D) de la perfusion et dont la surface délimitée en dessous dudit niveau est donc fonction du volume (V) de liquide restant à perfuser.

10. Procédé selon la revendication 9, caractérisé en ce qu'on affiche une représentation graphique (G) d'un goutte-à-goutte simulant la chute réelle des gouttes, avec une cadence fonction du débit instantané mesuré.

11. Procédé selon l'une quelconque des revendications 9 et 10, caractérisé en ce qu'on affiche le débit instantané sur un vu-mètre linéaire (VM) bordé à ses extrémités par les valeurs minimale et maximale prédéterminées du débit.

12. Procédé selon la revendication 11, caractérisé en ce que ledit vu-mètre est disposé verticalement sous la représentation graphique dudit récipient (R) et se présente sous la forme d'une série d'éléments consécutifs (52) symbolisant des gouttes, les éléments compris entre le débit minimal prédéterminé et le débit instantané mesuré étant affiché sous un aspect différent de ceux compris entre le débit instantané mesuré et le débit maximal prédéterminé.

13. Procédé selon l'une quelconque des revendications 9 à 12, caractérisé en ce qu'on divise ledit écran de contrôle en plusieurs fenêtres verticales (F1, F2, F3, F4) affectées chacune à l'affichage de données d'identification et des grandeurs représentatives de l'état d'un poste de perfusion déterminé.

14. Procédé selon la revendication 13, caractérisé en ce que lesdites données d'identification affichées dans une fenêtre comprennent le code d'identification propre au poste de perfusion correspondant ainsi que des informations relatives au patient (identité, localisation, etc ...) sous perfusion.

15. Système pour la mise en oeuvre du procédé selon l'une quelconque des revendications 1 à 14, caractérisé en ce qu'il comprend une ligne électrique (4) de transmission d'informations numériques, au moins un poste de perfusion (P1, P2 ...Pn) comportant un débitmètre (3) et un module (M1, M2 ...Mn) de codage et de transmission sous forme numérique sur la ligne (4) de l'information de débit instantané délivrée par le débitmètre (3), une unité centrale (UC) de traitement de ladite information, connectée à ladite ligne (4) par l'intermédiaire d'un module de décodage (Muc), et au moins un écran de contrôle pour l'affichage de données d'identification et de fonctionnement de chaque poste de perfusion sous surveillance.

16. Système selon la revendication 15, caractérisé en ce que l'unité centrale (UC) comprend un processeur (5) associé à des touches de fonction (7-12), à un clavier numérique (13), à des moyens d'affichage (14) par l'intermédiaire d'un circuit (15) de génération de graphismes, et à des moyens (16) de génération d'alarmes.

17. Système selon l'une quelconque des revendications 15 et 16, caractérisé en ce que le débitmètre (3) est un compte-gouttes.

18. Système selon l'une quelconque des revendications 15 et 16, caractérisé en ce que, dans le cas où le débit de la perfusion est commandé à chaque poste par une pompe asservie, le module (M1-Mn) associé à chaque poste (P1-Pn) comporte des moyens de décodage et le module (Muc) associé à l'unité centrale (UC) comporte des moyens de codage d'informations numériques pour la commande de chaque pompe à partir de l'unité centrale.

19. Système selon l'une quelconque des revendications 15 à 18, caractérisé en ce que ladite ligne électrique (4) est une ligne de transmission banalisée du réseau de distribution d'énergie électrique.

20. Système selon l'une quelconque des revendications 15 à 19, caractérisé en ce que chaque récipient de perfusion (1) comporte un capteur de niveau adapté pour délivrer un signal de déclenchement d'alarme en réponse à la détection d'un niveau minimal de liquide.

21. Système selon l'une quelconque des revendications 15 à 20, caractérisé en ce qu'il comprend un dispositif d'alarme à distance comportant un émetteur haute-fréquence (19a) connecté à l'unité centrale et une récepteur portable (19b), ledit récepteur présentant des moyens de génération d'un signal d'alarme en cas de détection d'une anomalie de fonctionnement d'un poste de perfusion par l'unité centrale.

22. Système selon la revendication 21, caractérisé en ce que ledit récepteur (19b) comprend un transducteur acoustique et des moyens d'affichage d'une donnée d'identification d'un poste de perfusion présentant une anomalie de fonctionnement.

23. Système selon la revendication 17, caractérisé en ce qu'à l'unité centrale est associé un moyen (60) d'étalonnage du volume unitaire des gouttes comptées par le compte-gouttes.

FIG.:1

FIG.:2

0233115

1_3

FIG.:3

0233115

**FIG.:5**

| F1 | F2 | 3_3 F3 | F4 |
|---|---|---|---|
| VOIE 1 | PERF. N°2 Cbre N°12 V1 = D1 = liquide ? | VOIE 3 | VOIE 4 |
| NON UTILISÉE | | NON UTILISÉE | NON UTILISÉE |

V1

V=
D=

½

R

G

0
△ prévu

VM

35 △ ↑ 80
Instantané

**FIG.:6**

R

V=
D=

△ min.

52 — VM

△ prévu

△ instantané

△ max.

30 → **INTERRUPTION** 31

**INTERROGATION POSTE DE PERFUSION** 32

34 **MESSAGE ERREUR**

NON — **TRANSMISSION ?** 33 — OUI

**FIG.:4**

**MISE À JOUR PARAMÈTRES** — 35

37 OUI — **ALARMES ?** 36 — NON

**MESSAGE ALARME**

OUI — **ALARME ROUGE ?** — NON

39 **DRAPEAU**

38

40 — **RETOUR PROGRAMME PRINCIPAL**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| Y | DE-A-3 329 977 (BRAUN MELSUNGEN)<br>* Figures 1,3a; revendications 1-6; page 12, lignes 9-20; page 13, lignes 1-31 * | 1-20 | A 61 M 5/14 |
| Y,D | WO-A-8 503 395 (S. RINUY)<br>* Abrégé * | 1-20 | |
| A | GB-A-2 039 083 (CRONE et al.)<br>* Page 1, ligne 58 - page 2, ligne 55 * | 1-23 | |
| A | FR-A-2 163 893 (S.A.T.A.S.)<br>* Page 2, lignes 12-18; page 2, ligne 36 - page 3, ligne 1; figure 1 * | 1,15 | **DOMAINES TECHNIQUES RECHERCHES (Int. Cl.4)** |
| A | DE-A-3 303 102 (SIEMENS)<br><br>* Page 5, lignes 4-8,16-24; page 7, lignes 3-7,20-24; page 9, lignes 21-24; page 9, ligne 33 - page 10, ligne 2; page 7, lignes 7-11; figures * | 1-6,15 -20 | A 61 M<br>G 01 F |

--- -/-

Le présent rapport de recherche a été établi pour toutes les revendications

| Lieu de la recherche | Date d'achèvement de la recherche | Examinateur |
|---|---|---|
| LA HAYE | 27-04-1987 | RAKOWICZ, J.M. |

| Catégorie | Citation du document avec indication, en cas de besoin, des parties pertinentes | Revendication concernée | CLASSEMENT DE LA DEMANDE (Int. Cl.4) |
|---|---|---|---|
| A | US-A-4 553 958 (LECOCQ)<br><br>* Figures 2A,2B; colonne 4, lignes 14-37,53-56; colonne 5, lignes 3-23; colonne 8, lignes 48-58; colonne 9, lignes 18-20,38-47 * | | |
| A | EP-A-0 013 334 (DOEHN)<br><br>* Page 4, ligne 19 - page 5, ligne 10; figure 1 * | | |

---

DOMAINES TECHNIQUES RECHERCHES (Int. Cl.4)

Le présent rapport de recherche a été établi pour toutes les revendications

| Lieu de la recherche | Date d'achèvement de la recherche | Examinateur |
|---|---|---|
| LA HAYE | 27-04-1987 | RAKOWICZ,J.M. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant